# EUROPEAN PATENT SPECIFICATION

(11) **EP 4 094 774 B1**
(45) Date of publication and mention of the grant of the patent: **08.10.2025**
(21) Application number: 21757541.4
(22) Date of filing: 19.02.2021
(51) Int. Cl.: A61K 38/21, A61P 31/12, A61P 31/16, A61P 11/00, A61P 31/14

(54) **USE OF INTERFERON IN PREPARING DRUG FOR PREVENTING CORONAVIRUS INFECTION**
VERWENDUNG VON INTERFERON ZUR HERSTELLUNG EINES ARZNEIMITTELS ZUR VORBEUGUNG VON CORONAVIRUS-INFEKTIONEN
UTILISATION D'INTERFÉRON DANS LA PRÉPARATION D'UN MÉDICAMENT POUR LA PRÉVENTION D'UNE INFECTION À CORONAVIRUS

(30) Priority: 21.02.2020 CN 202010108085; 07.05.2020 CN 202010378594
(43) Date of publication of application: 30.11.2022
(73) Proprietor: Shanghai Ganyi Medical Biotechnology Inc., Shanghai 200120 (CN); Meng, Zhongji, Hubei 442012 (CN); Ll, Hai, Shanghai 200035 (CN)
(72) Inventor: MENG, Zhongji, Hubei 442012 (CN); LI, Hai, Shanghai 200035 (CN)
(74) Representative: De Clercq & Partners
(86) International application number: PCT/CN2021/076904
(87) International publication number: WO 2021/164740

(56) References cited:
- WO-A2-2010/120511
- CN-A- 1 535 724
- CN-A- 1 777 438
- CN-A- 111 671 886
- CN-B- 1 927 389
- CN-B- 102 416 165
- US-A1- 2005 002 901
- ANONYMOUS: "The Effect of Recombinant Human Interferon Alpha Nasal Drops to Prevent COVID-19 Pneumonia for Medical Staff in an Epidemic Area | Bentham Science", 29 April 2021 (2021-04-29), XP093001224, Retrieved from the Internet <URL:http://www.eurekaselect.com/article/115517> [retrieved on 20221123]
- TURNER R B ET AL: "PREVENTION OF EXPERIMENTAL CORONAVIRUS COLDS WITH INTRANASAL ALPHA-2B INTERFERON", JOURNAL OF INFECTIOUS DISEASES, UNIVERSITY OF CHICAGO PRESS, US, vol. 154, no. 3, 1 September 1986 (1986-09-01), pages 443 - 447, XP008037795, ISSN: 0022-1899
- HAAGMANS BART L ET AL: "Pegylated interferon-[alpha] protects type 1 pneumocytes against SARS coronavirus infection in macaques", NATURE MEDICINE, NATURE PUBLISHING GROUP US, NEW YORK, vol. 10, no. 3, 22 February 2004 (2004-02-22), pages 290 - 293, XP037065941, ISSN: 1078-8956, [retrieved on 20040222], DOI: 10.1038/NM1001
- ABDO-CUZA ANSELMO ET AL: "Safety and efficacy of intranasal recombinant human interferon alfa 2b as prophylaxis for COVID-19 in patients on a hemodialysis program", vol. 7, no. 1, 29 September 2020 (2020-09-29), pages e05 - e05, XP093001226, ISSN: 2423-6438, Retrieved from the Internet <URL:https://jrenendo.com/PDF/jre-7-e05.pdf> DOI: 10.34172/jre.2021.05
- KUNLING SHEN ET AL.: "Diagnosis, Treatment, and Prevention of 2019 Novel Coronavirus Infection in Children: Experts’ Consensus Statement", WORLD JOURNAL OF PEDIATRICS, vol. 16, no. 3, 7 February 2020 (2020-02-07), XP037176937, DOI: 10.1007/s12519-020-00343-7
- YU, DEXIAN ET AL.: "A Field Trial of Recombinant Human Interferon α-2b for Nasal Spray to Prevent SARS and Other Respiratory Viral Infections", CHINESE JOURNAL OF EXPERIMENTAL AND CLINICAL VIROLOGY, vol. 19, no. 3, 30 September 2005 (2005-09-30), pages 216 - 219, XP055840338

## Description

### Technical Field

The present disclosure relates to the technical field of biomedicine, and in particular, to interferon for use in preventing coronavirus infection, wherein the coronavirus is SARS-CoV-2 infected through droplet-nose-respiratory tract, the interferon is IFN-α1b, and the interferon is administered nasally.

### Background Art

Novel coronavirus (2019-nCoV) is a new type of virus that broke out in December 2019. The results of nucleotide sequencing analysis of the virus shows that the virus is a coronavirus. The disease caused by infection of the novel coronavirus in the human body is named COVID-19 by the WHO, in which CO stands for corona, VI stands for virus, D stands for disease, and 19 stands for 2019, according to the statement of the WHO. At present, the virus has been officially named "SARS-CoV-2" (Severe Acute Respiratory Syndrome Coronavirus 2) by the International Committee on Taxonomy of Viruses.

The main route of transmission of the novel coronavirus is through droplets. The virus is inhaled into the nasal cavity along with air, and finally infects the lung as a main target organ, causing the virus to be abundantly present in alveolar epithelial cells. The novel coronavirus infects healthy people and causes the novel coronavirus disease (COVID-19), of which the novel coronavirus pneumonia is a main disease type of COVID-19. The main clinical manifestations of patients with pneumonia caused by novel coronavirus infection include fever, fatigue, and dry cough. Upper respiratory symptoms such as nasal congestion and runny nose are not common. Novel coronavirus pneumonia is divided into mild cases and severe cases according to severity. About half of the patients often develop dyspnea a week after infection, which may rapidly progress to acute respiratory distress syndrome, septic shock, and difficult-to-correct metabolic acidosis, and coagulation disorders in severe cases. The severe and critically ill patients may have moderate to low fever or even no obvious fever during the course of the disease. Some patients have onset of mild symptoms and may have no fever, and most of them recover after one week. Most patients have a good prognosis, and a small number of patients are critically ill and even die.

Severe viral pneumonia may cause a cytokine storm, thus leading to multiple organ failure. The novel coronavirus, which is very infectious, is reported in different literatures to have a basic reproduction number (R0) of 2.2 to 3.7, where the basic reproduction number (R0) represents the infectivity of the virus, and R0>1 indicates that the virus is highly infectious and may lead to a large outbreak. It was reported that different numbers of medical workers who first came into contact with COVID-19 patients were infected in the countries around the world. An Pan *et al.* reported that 1496 medical workers were infected with novel coronavirus, accounting for 4.6% of the COVID-19 patients in the entire region. In February 2020, epidemiology group of emergency response mechanism for novel coronavirus pneumonia in Chinese Center for Disease Control and Prevention published a paper on the analysis of epidemiological characteristics of novel coronavirus pneumonia in the Chinese Journal of Epidemiology. This paper reported that 3019 medical workers were infected from December 8, 2019 to February 11, 2020, among which there are 1716 confirmed cases. (Reference 1, page 149, left column, paragraph 3). In addition, reports about infection status nationwide and among medical workers in Italy showed that 2026 health care workers were infected among 22512 COVID-19 patients in Italy (Reference 2).

In addition, it was found that some asymptomatic persons, the so-called healthy carriers, existed among the 2019-nCoV infected patients. Asymptomatic persons are virally contagious, and may also develop novel coronavirus disease. The rate of iatrogenic infection of medical workers exposed to 2019-nCoV infected patients is about 3-5% under the condition of strict physical protection. However, there is currently neither drug that is confirmed to effectively treat the novel coronavirus infection, nor drug that can effectively prevent infection of the novel coronavirus in healthy people. At this stage, the prevention of infection of the novel coronavirus in healthy people mainly relies on physical barriers such as masks and isolation clothing.

Reference 1: Epidemiology group of emergency response mechanism for novel coronavirus pneumonia, Chinese Center for Disease Control and Prevention. Analysis of Epidemiological characteristics of novel coronavirus pneumonia [J]. Chinese Journal of Epidemiology, 2020, 41 (2): 145-151.

Reference 2: Edward L, Karen B. Coronavirus Disease 2019 (COVID-19) in Italy[J]. JAMA, 2020, 323(14):-.

Kunling Shen et al. "Diagnosis, Treatment, and Prevention of 2019 Novel Coronavirus Infection in Children: Experts Consensus Statement", World Journal of Pediatrics, vol. 16, no. 3, 2020-02-07, relates to treating, and preventing 2019-nCoV infections and specifically discloses that interferon-a can reduce viral load in early stages of infection. The use of an interferon-a nebulizer and an interferon-a 2b spray is recommended.

### Summary

The present disclosure provides interferon for use in preventing SARS-CoV-2 infection, wherein the SARS-CoV-2 is infected through droplet-nose-respiratory tract, so as to solve the technical problem that there is currently neither effective drug for treating SARS-CoV-2 infection, nor effective drug and approach for preventing SARS-CoV-2 infection in a healthy people and preventing novel coronavirus diseases. The invention is defined in the claims.

The interferon is IFN-α1b.

In one or more embodiments, the interferon is a natural interferon or a recombinant human interferon, and is for example a recombinant human interferon.

In one or more embodiments, the interferon in the present disclosure may be a long-acting interferon.

In one or more embodiments, the long-acting interferon in the present disclosure is PEG-IFN.

The interferon is administered nasally.

In one or more embodiments, the interferon is present in a dosage form that is prepared with the interferon as a pharmaceutically active ingredient. Preferably, the dosage form is a nasal formulation, and more preferably, the nasal formulation is a nasal drop or a nasal spray.

In one or more embodiments, the nasal formulation provided by the present disclosure may be a nasal drop, a nasal wash, a nasal spray, or an absorbent applied to nasal surface skin, and is for example a nasal drop or a nasal spray.

In one or more embodiments, the nasal formulation provided by the present disclosure includes a buffer, and the buffer is normal saline, phosphate buffer, citrate buffer, glucose saline (5% glucose injection), sodium bicarbonate buffer, acetate buffer, Tris-hydrochloric acid buffer, and is for example citrate buffer.

In one or more embodiments, the nasal formulation further includes other pharmaceutically acceptable carrier or excipient.

In one or more embodiments, the pharmaceutically acceptable carrier or excipient is one or more selected from the group consisting of mucosal absorption enhancers, protectants, and bacteriostatic agents.

In one or more embodiments, the concentration of the mucosal absorption enhancer in the carrier or excipient is 4-28% (v/v), preferably 6-14% (v/v), and more preferably 10% (v/v).

In one or more embodiments, the concentration of the protectant in the carrier or excipient is 0.3-2.5% (w/v), preferably 0.8-1.6% (w/v), and more preferably 1.2% (w/v).

In one or more embodiments, the concentration of the bacteriostatic agent in the carrier or excipient is 0.2-0.6% (w/v), preferably 0.25-0.4% (w/v), and more preferably 0.3% (w/v).

In one or more embodiments, the concentration of the mucosal absorption enhancer in the carrier or excipient is 4-28% (v/v), preferably 4-15% (v/v), and more preferably 5% (v/v).

In one or more embodiments, the concentration of the protectant in the carrier or excipient is 0.3-2.5% (w/v), preferably 0.3-1.0% (w/v), and more preferably 0.5% (w/v).

In one or more embodiments, the concentration of the bacteriostatic agent in the carrier or excipient is 0.2-0.6% (w/v), preferably 0.45-0.6% (w/v), and more preferably 0.5% (w/v).

In one or more embodiments, the mucosal absorption enhancer is a surfactant, cyclodextrin and its derivatives, mannitol, a phospholipid, a peptide and a proteolytic enzyme inhibitor, glycyrrhetinic acid and its derivatives, a metal ion chelating agent, and is preferably mannitol; the protectant is one or more of human albumin, artificial plasma, erythropoietin, brain-derived neurotrophic factor, nerve growth factor, epidermal growth factor, fibroblast growth factor, basic fibroblast growth factor, insulin-like growth factor 1, hyaluronidase, neuregulin, leukemia inhibitory factor, interleukin, interferon-like active substance, tumor necrosis factor, glial growth factor, growth differentiation factor, and nerve growth-related protein, and is preferably human albumin; the bacteriostatic agent is one or a combination of two or more of sorbic acid, potassium sorbate, benzoic acid, and parabens, and is preferably sodium benzoate; and the buffer is one or more of phosphate buffer, citrate buffer, glucose saline (5% glucose injection), sodium bicarbonate buffer, acetate buffer, and Tris-HCl buffer.

In the nasal formulation provided in one or more embodiments of the present disclosure, the final concentration of the interferon is 1000 U/ml to 3000000 U/ml. The interferon in the above concentration range can effectively inhibit replication and activity of virus in nasal epithelial cells. In accordance with inhibitory effect and pharmaceutical cost, the concentration of the interferon is preferably 2000 U/ml to 10000 U/ml, for example, 3000 U/ml, 4000 U/ml, 5000 U/ml, 6000 U/ml, 7000 U/ml, or 8000 U/ml.

In one or more embodiments, the concentration of the interferon is preferably 5000 U/ml to 12000 U/ml, for example, 6000 U/ml, 7000 U/ml, or 8000 U/ml.

The final concentration is the concentration of interferon in the formulation finally prepared.

The nasal formulation prepared in the present disclosure has a pH value of 5.0-8.0.

The present disclosure also provides a drug for use in preventing SARS-CoV-2 infection, in which the drug includes the interferon as defined in claim 1 and is a nasal formulation and characterized in that the interferon is administered nasally.

The drug provided in one or more embodiments of the present disclosure further includes a pharmaceutically acceptable carrier or excipient to prepare it into a nasal formulation.

In one or more embodiments, the nasal formulation provided by the present disclosure may be a nasal drop, a nasal wash, a nasal spray, or an absorbent applied to nasal surface skin, and is for example a nasal drop or a nasal spray.

In one or more embodiments, the nasal formulation provided by the present disclosure includes a buffer, and the buffer is normal saline, phosphate buffer, citrate buffer, glucose saline (5% glucose injection), sodium bicarbonate buffer, acetate buffer, Tris-HCl buffer, and is for example normal saline.

In one or more embodiments, the pharmaceutically acceptable carrier or excipient is one or more selected from the group consisting of mucosal absorption enhancers, protectants, and bacteriostatic agents.

In one or more embodiments, the concentration of the mucosal absorption enhancer in the carrier or excipient is 4-28% (v/v), preferably 6-14% (v/v), and more preferably 10% (v/v).

In one or more embodiments, the concentration of the protectant in the carrier or excipient is 0.3-2.5% (w/v), preferably 0.8-1.6% (w/v), and for example 1.2% (w/v).

In one or more embodiments, the concentration of the bacteriostatic agent in the carrier or excipient is 0.2-0.6% (w/v), preferably 0.25-0.4% (w/v), and for example 0.3% (w/v).

In one or more embodiments, the concentration of the mucosal absorption enhancer in the carrier or excipient is 4-28% (v/v), preferably 4-15% (v/v), and for example 5% (v/v).

In one or more embodiments, the concentration of the protectant in the carrier or excipient is 0.3-2.5% (w/v), preferably 0.3-1.0% (w/v), and for example 0.5% (w/v).

In one or more embodiments, the concentration of the bacteriostatic agent in the carrier or excipient is 0.2-0.6% (w/v), preferably 0.45-0.6% (w/v), and for example 0.5% (w/v).

In one or more embodiments, the mucosal absorption enhancer is a surfactant, cyclodextrin and its derivatives, mannitol, a phospholipid, a peptide and a proteolytic enzyme inhibitor, glycyrrhetinic acid and its derivatives, a metal ion chelating agent, and is preferably mannitol; the protectant is one or more of human albumin, artificial plasma, erythropoietin, brain-derived neurotrophic factor, nerve growth factor, epidermal growth factor, fibroblast growth factor, basic fibroblast growth factor, insulin-like growth factor 1, hyaluronidase, neuregulin, leukemia inhibitory factor, interleukin, interferon-like active substance, tumor necrosis factor, glial growth factor, growth differentiation factor, and nerve growth-related protein, and is preferably human albumin; the bacteriostatic agent is one or a combination of two or more of sorbic acid, potassium sorbate, benzoic acid, and parabens, and is preferably sodium benzoate; and the buffer is one or more of phosphate buffer, citrate buffer, glucose saline (5% glucose injection), sodium bicarbonate buffer, acetate buffer, and Tris-HCl buffer.

In the nasal formulation provided in one or more embodiments of the present disclosure, the final concentration of the interferon is 1000 U/ml to 3000000 U/ml in accordance with inhibitory effect and pharmaceutical cost, and the interferon in the above concentration range can effectively inhibit replication and activity of virus in nasal epithelial cells. In one or more embodiments, the concentration of interferon is 2000 U/ml to 10000 U/ml, for example, 2200 U/ml, 2500 U/ml, 2800 U/ml, 3000 U/ml, 3200 U/ml, 3500 U/ml, 3800 U/ml, 4000 U/ml, 4200 U/ml, 4500 U/ml, 4800 U/ml, 5000 U/ml, 5200 U/ml, 5500 U/ml, 5800 U/ml, 6000 U/ml, 6200 U/ml, 6500 U/ml, 6800 U/ml, 7000 U/ml, 7200 U/ml, 7500 U/ml, 7800 U/ml, 8000 U/ml, 8200 U/ml, 8500 U/ml, 8800 U/ml, 9000 U/ml, 9200 U/ml, 9500 U/ml, or 9800 U/ml.

In one or more embodiments, the concentration of interferon is preferably 5 000 U/ml to 12000 U/ml, for example, 6000 U/ml, 7000 U/ml, or 8000 U/ml.

The final concentration is the concentration of the interferon in the formulation finally prepared.

The nasal formulation prepared in one or more embodiments of the present disclosure has a pH value of 5.0-8.0.

The nasal formulation may be prepared by directly combining interferon with the pharmaceutically acceptable carrier or excipient, or mixing interferon with the buffer to prepare an interferon buffer and then combining the interferon buffer with the pharmaceutically acceptable carrier or excipient.

Advantageous effects of the present disclosure are as follows. The drug and composition of the present disclosure achieve unexpected technical effects, and no case of iatrogenic infection of SARS-CoV-2 occurs among the users within 2 weeks. Therefore, the technical solution of the present disclosure can effectively prevent SARS-CoV-2 infection, significantly reduce the infection rate of human populations and medical workers, and avoid the spread of the epidemic. The nasal formulation provided in the present disclosure can effectively maintain a relatively high concentration of interferon in the nose, and is easy to prepare, convenient to use and stable in quality. The nasal formulation can be effectively applied for the prevention of SARS-CoV-2 virus infection.

### Detailed Description of the Embodiments

The present disclosure may be more readily understood in reference to following detailed description of some embodiments of the present disclosure and the examples included therein.

Before further description of the present disclosure, it should be understood that the present disclosure is not limited to the particular embodiments described, because such embodiments are necessarily in various manners. It should also be understood that the terms used in this specification are intended to illustrate particular embodiments and not of limitation, because the scope of the present disclosure is defined by the appended claims merely.

Unless otherwise defined herein, scientific and technical terms used in the present disclosure shall have the meanings commonly understood by one of ordinary skill in the art. The meaning and scope of terms should be clear, however, the definitions provided herein take precedence over any dictionary or foreign definitions in the event of any potential ambiguity. In the present application, the use of the terms "comprise/include/contain" and their other forms is non-limiting.

In order that the present disclosure can be more readily understood, selected terms are defined below.

As used herein, the "coronavirus" generally refers to a member of the family Coronaviridae, particularly a virus species of the subfamily Coronaviridae in the family Coronaviridae, and is a positive-sense RNA virus that infects humans and animals and causes respiratory, gastrointestinal, or neurological disease.

The "novel coronavirus" is a novel virus that broke out around December 2019, and has been officially named by the World Health Organization as SARS-CoV-2. "2019-novel coronavirus", "2019-nCoV", and "Severe Acute Respiratory Syndrome Coronavirus 2" are aliases for the virus and can be used interchangeably.

The "SARS-CoV-2 infection" is an infectious disease caused by the novel coronavirus (SARS-CoV-2), including, but not limited to, "novel coronavirus pneumonia". The World Health Organization named it "Corona Virus Disease 2019", abbreviated as "COVID-19", and National Health Commission of the PRC named it "Novel Coronavirus Pneumonia" and "NCP".

The use in the "prevention of SARS-CoV-2 infection" includes use in the prevention of novel coronavirus from infecting healthy people, and refers to preventive measures taken to protect or prevent individuals who have not yet been infected with the virus from infection.

Applicable targets (healthy people mentioned above) for the prevention of SARS-CoV-2 infection described in the present disclosure are preferably healthy people who are negative for novel coronavirus nucleic acid detection or novel coronavirus immunoglobulin.

The medical workers in the present disclosure refer to doctors, nurses or paramedics who work in the hospitals in SARS-CoV-2 endemic areas, and include the following two groups: 1) medical workers who work in non-isolation wards or non-fever clinics and are not explicitly and directly exposed to patients diagnosed with SARS-CoV-2; and 2) medical workers in direct contact with patients diagnosed with SARS-CoV-2, for example, medical workers who involve in the diagnosis and treatment of patients diagnosed with SARS-CoV-2 in isolation wards, or medical workers who work in fever clinics.

The applicable objects for the prevention of novel coronavirus diseases are preferably SARS-CoV-2 infected people who have been infected with SARS-CoV-2 but have no obvious symptoms.

Not according to the claimed invention is the use for "novel coronavirus disease caused by SARS-CoV-2 infection", which includes preventing people who have been infected with novel coronavirus from developing the "novel coronavirus disease" or other severe infection consequences.

The race of the healthy people generally refers to all races, including but not limited to the yellow race, the white race, the black race, the red race, or the mixed race thereof.

Interferons are divided into two types according to their effects: type I interferons and type II interferons. Type I interferons contain α interferon (IFN-α) and β interferon (IFN-β), and type II interferons contain γ interferon (IFN-γ). According to the antigenicity, interferons are also divided into α, β, and γ interferons. Le interferon is α-interferon (IFN-α), F interferon is β-interferon (IFN-β), and T-interferon is γ-interferon (IFN-γ). α, β, and γ interferons can be divided into several subtypes according to their amino acid sequences of each subtype. The subtypes of α-interferon include IFN-α1, IFN-α2, IFN-α3 (or IFN-αA, IFN-αB, IFN-αC), and IFN-ω.

The term "interferon" includes the above-mentioned type I interferon and type II interferon, as well as natural interferons, artificial recombinant interferons, and their subtypes, and the above-mentioned subtypes and IFN-α1b, IFN-a2b, IFN-a1a, IFN-a2a, PEG-IFN-α2b, and the like.

The natural interferon described in the present disclosure refers to the interferon obtained through extraction from human tissue.

The recombinant interferon described in the present disclosure is obtained by gene recombination technology, which involves cloning an interferon gene into a vector, transferring the vector into a host cell and culturing it, and collecting or purify the culture to obtain the interferon.

The present disclosure also relates to an isolated nucleic acid molecule, which is DNA or RNA encoding the interferon protein as described above. The nucleic acid or its fragment of the present disclosure may be inserted into a suitable vector to form a cloning vector or expression vector carrying the nucleic acid fragment of the present disclosure. This new vector is also a part of the present disclosure. The vectors may include plasmids, phages, cosmids, minichromosomes, or viruses, as well as naked DNA that is transiently expressed in specific cells only. The cloning and expression vectors of the present disclosure are capable of spontaneous replication and thus can provide high copy numbers for high level expression or high level replication of subsequent cloning. The expression vector may include a promoter for driving expression of the nucleic acid fragment of the present disclosure, an optional nucleic acid sequence encoding a signal peptide that enables secretion or integration of the peptide expression product onto a membrane, the nucleic acid fragment of the present disclosure, and an optional nucleic acid sequence encoding a terminator. When the expression vector is manipulated in a production strain or cell line, the vector may or may not be integrated into the host cell genome upon introducing it into the host cell. The vector typically carries a replication site and a marker sequence capable of providing phenotypic selection in transformed cells.

The term "vector" refers to a molecule or reagent that includes the nucleic acid or its fragment of the present disclosure and is capable of carrying genetic information and delivering it into a cell. Typical vectors include plasmids, viruses, phages, cosmids, and minichromosomes. The cloning vector may contain a selectable marker and an origin of replication compatible with the cell type specified by the cloning vector, and the expression vector may contain a regulatory element necessary to affect expression in a specified target cell.

The host cells that can be used to express the above vectors include prokaryotic cells and eukaryotic cells. The prokaryotic cells include, but are not limited to, *E. coli,* and the eukaryotic cells include, but are not limited to, yeast, insect cells, and mammal cells. The host cells may also include many immortalized cell lines. In some aspects of the present disclosure, the interferon may be modified by polyethylene glycol (PEG) to obtain a long-acting interferon. Alternatively, long-acting interferons that are commercially available can also be used.

In one or more embodiments, the nasal formulation of the present disclosure may also include a pharmaceutically acceptable diluent, excipient, or carrier. It should be understood that the pharmaceutically acceptable diluent, excipient, or carrier is compatible with the active ingredient and is not detrimental to the individual to which it is administered. The diluent may include, but is not limited to, normal saline, potassium chloride, potassium hydrogen phosphate, potassium dihydrogen phosphate, sodium sulfate, sodium chloride, potassium hydroxide, or a combination thereof. The diluents described above can be used to adjust the osmotic pressure and pH value and decrease/increase consistency or solubility of the drug of the present disclosure.

The carrier may include, but is not limited to, absorption enhancers, emulsifiers, protectants, alcoholic liquids, glycerol, polysorbates, or a combination thereof. The above-mentioned alcoholic liquid may be, but not limited to, a monohydric alcohol or a polyhydric alcohol.

In some examples, the excipient may be an antioxidant, a colorant, a bacteriostatic agent, or a combination thereof.

The drug of the present disclosure may also be used in conjunction with other supplements or prepared into other compositions.

The embodiments of the present disclosure will be described in detail below with reference to the examples, but those skilled in the art will understand that the following examples are only used to illustrate the present disclosure and should not be deemed to limit the scope of the present disclosure. The examples for which specific conditions are not indicated are carried out according to the conventional conditions or the conditions recommended by the manufacturers. The used reagents or instruments for which the manufacturers are not indicated are all conventional products that can be purchased from the market.

### Example 1 Preparation of recombinant human interferon formulation

Formulation 1: Recombinant human interferon α1b (manufactured by Beijing Tri-Prime Gene Pharmaceutical Co., Ltd., SFDA approval number S20010007, trade name: Hapgen) 2000 U/ml, the buffer is normal saline (0.9% sodium chloride injection).

Formulation 2: Recombinant human interferon α1b (manufactured by Beijing Tri-Prime Gene Pharmaceutical Co., Ltd., SFDA approval number S20010007, trade name: Hapgen) 3000 U/ml, the buffer is normal saline (0.9% sodium chloride injection).

Formulation 3: Recombinant human interferon α1b (manufactured by Beijing Tri-Prime Gene Pharmaceutical Co., Ltd., SFDA approval number S20010007, trade name: Hapgen) 4000 U/ml, the buffer is normal saline (0.9% sodium chloride injection)

Formulation 4: Recombinant human interferon α1b (manufactured by Beijing Tri-Prime Gene Pharmaceutical Co., Ltd., SFDA approval number S20010007, trade name: Hapgen) 5000 U/ml, the buffer is normal saline (0.9% sodium chloride injection).

Formulation 5: Recombinant human interferon α1b (manufactured by Beijing Tri-Prime Gene Pharmaceutical Co., Ltd., SFDA approval number S20010007, trade name: Hapgen) 8000 U/ml, glucose saline (5% glucose injection).

Formulation 6: Recombinant human interferon α1b (manufactured by Beijing Tri-Prime Gene Pharmaceutical Co., Ltd., SFDA approval number S20010007, trade name: Hapgen) 10000 U/ml, phosphate buffer.

Formulation 7: Recombinant human interferon α1b (manufactured by Beijing Tri-Prime Gene Pharmaceutical Co., Ltd., SFDA approval number S20010007, trade name: Hapgen) 5000 U/ml, citrate buffer, human albumin 0.3% (w/v), potassium sorbate 0.2% (w/v).

Formulation 8: Recombinant human interferon α1b (manufactured by Beijing Tri-Prime Gene Pharmaceutical Co., Ltd., SFDA approval number S20010007, trade name: Hapgen) 5000 U/ml, acetate buffer, epidermal growth factor 0.8% (w/v), benzoic acid 0.25% (w/v).

Formulation 9: Recombinant human interferon α1b (manufactured by Beijing Tri-Prime Gene Pharmaceutical Co., Ltd., SFDA approval number S20010007, trade name: Hapgen) 5000 U/ml, Tris-HCl buffer, erythropoietin 1.6% (w/v), paraben 0.4% (w/v).

Formulation 10: Recombinant human interferon α1b (manufactured by Beijing Tri-Prime Gene Pharmaceutical Co., Ltd., SFDA approval number S20010007, trade name: Hapgen) 5000 U/ml, normal saline, hyaluronidase 2.5% (w/v), sorbic acid 0.6% (w/v).

Formulation 11: Recombinant human interferon α1b (manufactured by Beijing Tri-Prime Gene Pharmaceutical Co., Ltd., SFDA approval number S20010007, trade name: Hapgen) 5000 U/ml, normal saline, human albumin 1.2% (w/v), sorbic acid 0.3% (w/v).

Formulation 12: Recombinant human interferon α1b (manufactured by Beijing Tri-Prime Gene Pharmaceutical Co., Ltd., SFDA approval number S20010007, trade name: Hapgen) 5000 U/ml, cyclodextrin 4% (w/v), human albumin 0.3% (w/v), potassium sorbate 0.2% (w/v), citrate buffer.

Formulation 13: Recombinant human interferon α1b (manufactured by Beijing Tri-Prime Gene Pharmaceutical Co., Ltd., SFDA approval number S20010007, trade name: Hapgen) 5000 U/ml, TW-80 (Tween 80) 6% (w/v), epidermal growth factor 0.8% (w/v), benzoic acid 0.25% (w/v), acetate buffer.

Formulation 14: Recombinant human interferon α1b (manufactured by Beijing Tri-Prime Gene Pharmaceutical Co., Ltd., SFDA approval number S20010007, trade name: Hapgen) 5000 U/ml, PEG-400 monooleate 14% (w/v), erythropoietin 1.6% (w/v), paraben 0.4% (w/v), Tris-HCl buffer.

Formulation 15: Recombinant human interferon α1b (manufactured by Beijing Tri-Prime Gene Pharmaceutical Co., Ltd., SFDA approval number S20010007, trade name: Hapgen) 5000 U/ml, phospholipid 28% (w/v), hyaluronidase 2.5% (w/v), sorbic acid 0.6% (w/v), normal saline.

Formulation 16: Recombinant human interferon α1b (manufactured by Beijing Tri-Prime Gene Pharmaceutical Co., Ltd., SFDA approval number S20010007, trade name: Hapgen) 5000 U/ml, TW-40 10% (w/v), human albumin 1.2% (w/v), sorbic acid 0.3% (w/v), normal saline.

Formulation 17 not according to the claimed invention: Recombinant human interferon α2b stock solution (manufactured by Tianjin Weiming Biopharmaceutical Co., Ltd. (formerly Tianjin Hualida Bioengineering Co., Ltd.)) 5000 U/ml, the buffer is normal saline (0.9% sodium chloride injection).

Formulation 18 not according to the claimed invention: Recombinant human interferon α2b stock solution (Tianjin Weiming Biopharmaceutical Co., Ltd. (formerly Tianjin Hualida Bioengineering Co., Ltd.)) 6000 U/ml, the buffer is normal saline (0.9% sodium chloride injection).

Formulation 19 not according to the claimed invention: Recombinant human interferon α2b stock solution (Tianjin Weiming Biopharmaceutical Co., Ltd. (formerly Tianjin Hualida Bioengineering Co., Ltd.)) 7000 U/ml, the buffer is normal saline (0.9% sodium chloride injection).

Formulation 20 not according to the claimed invention: Recombinant human interferon α2b stock solution (Tianjin Weiming Biopharmaceutical Co., Ltd. (formerly Tianjin Hualida Bioengineering Co., Ltd.)) 8000 U/ml, the buffer is normal saline (0.9% sodium chloride injection).

Formulation 21 not according to the claimed invention: Recombinant human interferon α2b stock solution (Tianjin Weiming Biopharmaceutical Co., Ltd. (formerly Tianjin Hualida Bioengineering Co., Ltd.)) 10000 U/ml, glucose saline (5% glucose injection).

Formulation 22 not according to the claimed invention: Recombinant human interferon α2b stock solution (Tianjin Weiming Biopharmaceutical Co., Ltd. (formerly Tianjin Hualida Bioengineering Co., Ltd.)) 12000 U/ml, phosphate buffer.

Formulation 23 not according to the claimed invention: Recombinant human interferon α2b stock solution (Tianjin Weiming Biopharmaceutical Co., Ltd. (formerly Tianjin Hualida Bioengineering Co., Ltd.)) 8000 U/ml, citrate buffer, human albumin 0.5% (w/v), sodium benzoate 0.5% (w/v).

Formulation 24 not according to the claimed invention: Recombinant human interferon α2b stock solution (Tianjin Weiming Biopharmaceutical Co., Ltd. (formerly Tianjin Hualida Bioengineering Co., Ltd.)) 8000 U/ml, acetate buffer, epidermal growth factor 0.8% (w/v), benzoic acid 0.25% (w/v).

Formulation 25 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, Tris-HCl buffer, erythropoietin 1.6% (w/v), paraben 0.4% (w/v).

Formulation 26 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, normal saline, hyaluronidase 2.5% (w/v), sorbic acid 0.6% (w/v).

Formulation 27 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, normal saline, human albumin 1.2% (w/v), sorbic acid 0.3% (w/v).

Formulation 28 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, cyclodextrin 4% (w/v), human albumin 0.3% (w/v), potassium sorbate 0.2% (w/v), citrate buffer.

Formulation 29 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, TW-80 6% (w/v), epidermal growth factor 0.8% (w/v), benzoic acid 0.25% (w/v), acetate buffer.

Formulation 30 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, PEG-400 monooleate 14% (w/v), erythropoietin 1.6% (w/v), paraben 0.4% (w/v), Tris-HCl buffer.

Formulation 31 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, phospholipid 28% (w/v), hyaluronidase 2.5% (w/v), sorbic acid 0.6% (w/v), normal saline.

Formulation 32 not according to the claimed invention: Recombinant human interferon α2b stock solution (Tianjin Weiming Biopharmaceutical Co., Ltd. (formerly Tianjin Hualida Bioengineering Co., Ltd.) 8000 U/ml, TW-80 0.004% (w/v), human albumin 0.5% (w/v), sodium benzoate 0.5% (w/v), mannitol 5% (w/v), citrate buffer.

The components in each formulation described above were mixed to prepare a recombinant human interferon nasal drop or nasal spray.

The original formulation of recombinant human interferon α1b used in the above formulations is commercially available, and its spray or injection can be used, diluted as required, and mixed with a pharmaceutically acceptable carrier or excipient to prepare nasal formulations of recombinant human interferon α1b in different concentrations in the present disclosure. The recombinant human interferon α2b is also commercially available. The recombinant human interferon α2b was diluted as required, and then mixed with a pharmaceutically acceptable carrier or excipient to prepare nasal formulations of recombinant human interferon α1b in different concentrations in the present disclosure. The concentrations of recombinant human interferon α1b and recombinant human interferon α2b given in Formulations 1-32 above were the final concentrations of interferon in Formulations 1-32.

### Example 2 Preventive effect of recombinant human interferon formulation (Formulation 2) for people outside isolation wards

As of February 5, 2020, a city in a 2019-nCoV epidemic area had a total of 353 patients diagnosed with 2019-nCoV infection. A considerable number of these patients were admitted to the infection isolation ward of Taihe Hospital Affiliated to Hubei University of Medicine, a third-grade class-A hospital in Shiyan City. Therefore, the interferon formulation described in the present disclosure was administrated to the medical workers who worked in a non-isolation ward or a non-fever clinic of Taihe Hospital, and were not explicitly and directly exposed to patients diagnosed with 2019-nCoV. Comparison was performed between infection status of these medical workers with those in third-grade class-A hospitals in Hubei epidemic area, to observe the preventive effect of the formulation on novel coronavirus infection.

In this example, about 2000 medical workers who worked outside of isolation wards and fever clinics and were not directly exposed to infected patients were each administrated with the α interferon nasal drop of Formulation 2 in Example 1 through nasal drip, 2-3 drops each time per nostril, four times a day, and observed for 14 days (from January 31 to February 13, 2020).

The observation results showed that, during the incubation period of the novel coronavirus, none of the subject medical workers in the non-isolation wards of Taihe Hospital was found to be infected with the virus, nor was there any case of the novel coronavirus disease (COVID-19).

### Example 3 Preventive effect of recombinant human interferon formulation (Formulation 28) not according to the claimed invention for people outside isolation wards

The interferon formulation described in the present disclosure was administrated to the medical workers who worked in a non-isolation ward or a non-fever clinic of a third-grade class-A hospital in Hubei Province, and were not explicitly and directly exposed to patients diagnosed with 2019-nCoV. Comparison was performed between infection status of these medical workers with those in third-grade class-A hospitals in Hubei epidemic area, to observe the preventive effect of the formulation on novel coronavirus infection.

In this example, about 200 medical workers who worked outside of isolation wards for the treatment of SRAS-CoV-2 patients and fever clinics and were not directly exposed to infected patients were each administrated with the α2b interferon nasal drop of Formulation 28 in Example 1 through nasal drip, 2-3 drops each time per nostril, four times a day, and observed for 14 days (from January 31 to February 13, 2020).

The observation results showed that, during the incubation period of the novel coronavirus, none of the subject medical workers in the non-isolation wards of Taihe Hospital was found to be infected with the virus, nor was there any case of the novel coronavirus disease (COVID-19).

### Example 4 Preventive effect of recombinant human interferon formulation (Formulation 2) for medical workers in isolation wards and fever clinics

The interferon formulation described in the present disclosure was administrated to the medical workers who worked in an isolation ward for the hospitalization of SRAS-CoV-2 patients in a third-grade class-A hospital in Hubei Province. Comparison was performed between infection status of these medical workers with those in third-grade class-A hospitals in Hubei epidemic area, to observe the preventive effect of the formulation on novel coronavirus infection.

In this example, more than 100 medical workers who were directly exposed to infected patients in isolation wards and more than 100 medical workers who worked in fever clinics were each administrated with the α interferon nasal drop of Formulation 2 in Example 1 through nasal drip, 2-3 drops each time per nostril, four times a day, and observed for 14 days (from January 31 to February 13, 2020).

The observation results showed that, during the incubation period of the novel coronavirus, none of the subject medical workers described above was found to be infected with the virus, nor was there any case of the novel coronavirus disease (COVID-19).

### Example 5 Preventive effect of recombinant human interferon formulation (Formulation 28) not according to the claimed invention for medical workers in isolation wards and fever clinics

The interferon formulation described in the present disclosure was administrated to the medical workers who worked in an isolation ward for the hospitalization of SRAS-CoV-2 patients in a third-grade class-A hospital in Hubei Province. Comparison was performed between infection status of these medical workers with those in third-grade class-A hospitals in Hubei epidemic area, to observe the preventive effect of the formulation on novel coronavirus infection.

In this example, more than 100 medical workers who were directly exposed to infected patients in isolation wards and more than 100 medical workers who worked in fever clinics were each administrated with the α2b interferon nasal drop of Formulation 28 in Example 1 through nasal drip, 2-3 drops each time per nostril, four times a day, and observed for 14 days (from January 31 to February 13, 2020).

The observation results showed that, during the incubation period of the novel coronavirus, none of the subject medical workers described above was found to be infected with the virus, nor was there any case of the novel coronavirus disease (COVID-19).

As the National Health Commission of the PRC currently recommends physical isolation measures for 2019-nCoV prevention, there are no official recommendations on prevention for 2019-nCoV by medicaments. The industry insiders believe that other third-grade class-A hospitals in the epidemic area of Hubei have not used any drug to prevent 2019-nCoV and the resulting COVID-19. From the outbreak of the epidemic to February 5, 2020, there are a total of 13 hospitals where 15 or more medical workers were iatrogenically infected. As of February 11, 2020, there were 3019 medical workers iatrogenically infected in Hubei Province (including confirmed, suspected and clinically diagnosed cases and asymptomatic infections, of which 1716 were confirmed cases). In addition, as described in the existing reports in Background Art, different numbers of medical workers who first came into contact with COVID-19 patients were infected in the countries around the world. An Pan *et al.* reported that 1496 medical workers were infected with novel coronavirus, accounting for 4.6% of the COVID-19 patients in the entire region (Reference 1, E5, Table). In February 2020, epidemiology group of emergency response mechanism for novel coronavirus pneumonia in Chinese Center for Disease Control and Prevention published a paper on the analysis of epidemiological characteristics of novel coronavirus pneumonia in the Chinese Journal of Epidemiology. This paper reported that 3019 medical workers were infected from December 8, 2019 to February 11, 2020, among which there are 1716 confirmed cases. (Reference 2, page 149, left column, paragraph 3). In addition, reports about infection status nationwide and among medical workers in Italy showed that 2026 health care workers were infected among 22512 COVID-19 patients in Italy (Reference 3). Thus, a certain proportion of medical workers for novel coronavirus pneumonia may be infected.

The iatrogenic infection proportion of medical workers in the third-grade class-A hospital hospitals in the epidemic area within 14 days after use of the α interferon nasal drop was zero, which was significantly lower than the infection rate of medical workers in the other third-grade class-A hospitals in the epidemic area. This unexpected result is of great significance for control of the infection.

Finally, it should be noted that the above examples are merely intended to illustrate the technical solutions of the present disclosure, but not to limit them. Although the present disclosure is described in detail with reference to the foregoing examples, those of ordinary skill in the art should understand that the technical solutions described in the foregoing examples may be modified, or equivalent substitutions may be made for some or all of the technical features; and these modifications or substitutions do not render the essence of the corresponding technical solutions to deviate from the technical scope of the examples of the present disclosure.

### Industrial Applicability

The drug and composition described in the present disclosure achieve unexpected technical effects, and no case of iatrogenic infection of SARS-CoV-2 occurs among the users within 2 weeks. Therefore, the technical solution of the present disclosure can effectively prevent SARS-CoV-2 infection, significantly reduce the infection rate of human populations and medical workers, and avoid the spread of the epidemic. The nasal formulation provided in the present disclosure can effectively maintain a relatively high concentration of interferon in the nose, and is easy to prepare, convenient to use and stable in quality. The nasal formulation can be effectively applied for the prevention of SARS-CoV-2 virus infection.

## Claims

1. Interferon for use in preventing coronavirus infection, wherein the coronavirus is SARS-CoV-2 infected through droplet-nose-respiratory tract, the interferon is IFN-α1b, and the interferon is administered nasally.

2. The interferon for use according to claim 1, wherein the interferon is a natural interferon or a recombinant human interferon, and preferably a recombinant human interferon.

3. The interferon for use according to claim 1 or 2, wherein the interferon is a long-acting interferon.

4. The interferon for use according to claim 1 or 2, wherein the interferon is present in a dosage form that is prepared with the interferon as a pharmaceutically active ingredient.

5. The interferon for use according to claim 4, wherein the dosage form is a nasal formulation, preferably the nasal formulation is a nasal drop or a nasal spray.

6. The interferon for use according to claim 5, wherein the nasal formulation comprises a buffer; and preferably, the buffer is normal saline, phosphate buffer, citrate buffer, glucose saline (5% glucose injection), sodium bicarbonate buffer, acetate buffer or Tris-hydrochloric acid buffer, and more preferably, citrate buffer; and
preferably, a final concentration of the interferon is 1000 U/ml to 3000000 U/ml, still preferably 2000 U/ml to 10000 U/ml or 5000 U/ml to 12000 U/ml, more preferably 3000 U/ml, 4000 U/ml, 5000 U/ml, 6000 U/ml, 7000 U/ml, or 8000 U/ml.

7. The interferon for use according to claim 6, wherein the nasal formulation further comprises another pharmaceutically acceptable carrier or excipient, preferably, the pharmaceutically acceptable carrier or excipient is one or more selected from the group consisting of a mucosal absorption enhancer, a protectant and a bacteriostatic agent; more preferably, the mucosal absorption enhancer is one or more selected from the group consisting of surfactants, cyclodextrins and derivatives thereof, mannitol, phospholipids, peptides and proteolytic enzyme inhibitors, glycyrrhetic acid and its derivatives and metal ion chelating agents, and is more preferably mannitol; the protectant is one or more selected from the group consisting of human albumin, artificial plasma, erythropoietin, brain-derived neurotrophic factor, nerve growth factor, epidermal growth factor, fibroblast growth factor, basic fibroblast growth factor, insulin-like growth factor 1, hyaluronidase, neuregulin, leukemia inhibitory factor, interleukin, interferon-like active substance, tumor necrosis factor, glial growth factor, growth differentiation factor, and nerve growth-related protein, and is more preferably human albumin; the bacteriostatic agent is one or more selected from the group consisting of sorbic acid, potassium sorbate, benzoic acid, parabens, and combined use of benzoic acid and potassium sorbate, and is more preferably sodium benzoate; preferably, a concentration of the mucosal absorption enhancer is 4-28% (v/v),
preferably 6-14% (v/v) or 4-15% (v/v), and more preferably 10% (v/v) or 5% (v/v);
preferably, a concentration of the protectant is 0.3-2.5% (w/v), preferably 0.8-1.6% (w/v) or 0.3-1.0% (w/v), and more preferably 1.2% (w/v) or 0.5% (w/v); and
preferably, a concentration of the bacteriostatic agent is 0.2-0.6% (w/v), preferably 0.25-0.4% (w/v) or 0.45-0.6% (w/v), and more preferably 0.3% (w/v) or 0.5% (w/v).

8. A drug for use in preventing coronavirus infection, wherein the coronavirus is SARS-CoV-2, **characterized in that** the drug is a nasal formulation and comprises the interferon according to claim 1 and **in that** the interferon is administered nasally; preferably the coronavirus is infected through droplet-nose-respiratory tract; and preferably, the drug further comprises the buffer described in claim 6 and/or the carrier or excipient described in claim 7.

## Patentansprüche

1. Interferon zur Verwendung beim Vorbeugen einer Coronavirus-Infektion, wobei es sich bei dem Coronavirus um SARS-CoV-2, dessen Infektion über Tröpfchen-Nase-Atemwege erfolgte, und bei dem Interferon um IFN-α1b handelt und das Interferon nasal verabreicht wird.

2. Interferon zur Verwendung nach Anspruch 1, wobei es sich bei dem Interferon um ein natürliches Interferon oder ein rekombinantes menschliches Interferon und bevorzugt um ein rekombinantes menschliches Interferon handelt.

3. Interferon zur Verwendung nach Anspruch 1 oder 2, wobei es sich bei dem Interferon um ein Interferon mit Langzeitwirkung handelt.

4. Interferon zur Verwendung nach Anspruch 1 oder 2, wobei das Interferon in einer Darreichungsform vorliegt, die mit dem Interferon als pharmazeutischem Wirkstoff hergestellt wird.

5. Interferon zur Verwendung nach Anspruch 4, wobei es sich bei der Darreichungsform um eine nasale Formulierung handelt, bei der es sich bevorzugt um Nasentropfen oder ein Nasenspray handelt.

6. Interferon zur Verwendung nach Anspruch 5, wobei die nasale Formulierung einen Puffer umfasst und es sich bei dem Puffer bevorzugt um normale Kochsalzlösung, Phosphatpuffer, Citratpuffer, Glucose-Salzlösung (5 %-Glucose-Injektion), Natriumhydrogencarbonat-Puffer, Acetatpuffer oder Tris-Salzsäure-Puffer und bevorzugter um Citratpuffer handelt und
eine Endkonzentration des Interferons bevorzugt 1000 U/ml bis 3000000 U/ml, weiter bevorzugt 2000 U/ml bis 10000 U/ml oder 5000 U/ml bis 12000 U/ml, bevorzugter 3000 U/ml, 4000 U/ml, 5000 U/ml, 6000 U/ml, 7000 U/ml oder 8000 U/ml beträgt.

7. Interferon zur Verwendung nach Anspruch 6, wobei die nasale Formulierung ferner einen weiteren pharmazeutisch unbedenklichen Träger oder Exzipienten umfasst, es sich bevorzugt bei dem pharmazeutisch unbedenklichen Träger oder Exzipienten bevorzugt um einen oder mehrere aus der Gruppe bestehend aus einem Schleimhautresorptionsverbesserer, einem Schutzstoff und einem Bakteriostatikum ausgewählte handelt, es sich bevorzugter bei dem Schleimhautresorptionsverbesserer um einen oder mehrere aus der Gruppe bestehend aus Tensiden, Cyclodextrinen und Derivaten davon, Mannit, Phospholipiden, Peptiden und Inhibitoren proteolytischer Enzyme, Glycyrrhetinsäure und ihren Derivaten und Metallionen-Chelatbildnern ausgewählte und bevorzugter um Mannit handelt, bei dem Schutzstoff um einen oder mehrere aus der Gruppe bestehend aus Humanalbumin, künstlichem Plasma, Erythropoetin, aus Gehirn stammendem neurotrophem Faktor, Nervenwachstumsfaktor, epidermalem Wachstumsfaktor, Fibroblasten-Wachstumsfaktor, basischem Fibroblasten-Wachstumsfaktor , insulinähnlichem Wachstumsfaktor 1, Hyaluronidase, Neuregulin, Leukämie-Hemmfaktor, Interleukin, interferonähnlichem Wirkstoff, Tumornekrosefaktor, Gliawachstumsfaktor, Wachstumsdifferenzierungsfaktor und nervenwachstumsbezogenem Protein ausgewählte und bevorzugter um Humanalbumin handelt und bei dem Bakteriostatikum um eines oder mehrere aus der Gruppe bestehend aus Sorbinsäure, Kaliumsorbat, Benzoesäure, Parabenen und kombinierter Verwendung von Benzoesäure und Kaliumsorbat ausgewählte und bevorzugter um Natriumbenzoat handelt, vorzugsweise eine Konzentration des Schleimhautresorptionsverbesserers 4-28 Vol.-%, bevorzugt 6-14 Vol.-% oder 4-15 Vol.-% und bevorzugter 10 Vol.-% oder 5 Vol.-% beträgt,
vorzugsweise eine Konzentration des Schutzstoffs 0,3-2,5 % (Gew./Vol.), bevorzugt 0,8-1,6 % (Gew./Vol.) oder 0,3-1,0 % (Gew./Vol.) und bevorzugter 1,2 % (Gew./Vol.) oder 0,5 % (Gew./Vol.) beträgt und
vorzugsweise eine Konzentration des Bakteriostatikums 0,2-0,6 % (Gew./Vol.), bevorzugt 0,25-0,4 % (Gew./Vol.) oder 0,45-0,6 % (Gew./Vol.) und bevorzugter 0,3 % (Gew./Vol.) oder 0,5 % (Gew./Vol.) beträgt.

8. Arzneistoff zur Verwendung beim Vorbeugen einer Coronavirus-Infektion, wobei es sich bei dem Coronavirus um SARS-CoV-2 handelt, **dadurch gekennzeichnet, dass** der Arzneistoff eine nasale Formulierung ist und das Interferon nach Anspruch 1 umfasst und dass das Interferon nasal verabreicht wird, vorzugsweise die Infektion des Coronavirus über Tröpfchen-Nase-Atemwege erfolgt und vorzugsweise der Arzneistoff ferner den in Anspruch 6 beschriebenen Puffer und/oder den in Anspruch 7 beschriebenen Träger oder Exzipienten umfasst.

## Revendications

1. Interféron destiné à être utilisé dans la prévention d'une infection à coronavirus, dans lequel le coronavirus est SARS-CoV-2 infecté par gouttelette-nez-voies respiratoires, l'interféron est l'IFN-α1B, et l'interféron est administré par voie nasale.

2. Interféron destiné à être utilisé selon la revendication 1, dans lequel l'interféron est un interféron naturel ou un interféron humain recombinant, et de préférence un interféron humain recombinant.

3. Interféron destiné à être utilisé selon la revendication 1 ou 2, dans lequel l'interféron est un interféron à action prolongée.

4. Interféron destiné à être utilisé selon la revendication 1 ou 2, dans lequel l'interféron est présent sous une forme posologique qui est préparée avec l'interféron comme un ingrédient pharmaceutiquement actif.

5. Interféron destiné à être utilisé selon la revendication 4, dans lequel la forme posologique est une formulation nasale, de préférence la formulation nasale est une goutte nasale ou un spray nasal.

6. Interféron destiné à être utilisé selon la revendication 5, dans lequel la formulation nasale comprend un tampon ; et de préférence, le tampon est une solution saline normale, un tampon phosphate, un tampon citrate, une solution saline de glucose (injection de glucose à 5 %), un tampon bicarbonate de sodium, un tampon acétate ou un tampon Tris-acide chlorhydrique, et plus préférablement un tampon citrate ; et
de préférence, la concentration finale de l'interféron est de 1 000 U/ml à 3 000 000 U/ml, de préférence encore de 2 000 U/ml à 10 000 U/ml ou de 5 000 U/ml à 12 000 U/ml, plus préférablement de 3 000 U/ml, 4 000 U/ml, 5 000 U/ml, 6 000 U/ml, 7 000 U/ml, ou 8 000 U/ml.

7. Interféron destiné à être utilisé selon la revendication 6, dans lequel la formulation nasale comprend en outre un autre support ou excipient pharmaceutiquement acceptable, de préférence, le support ou excipient pharmaceutiquement acceptable est un ou plusieurs choisis dans le groupe constitué par un activateur d'absorption muqueuse, un agent protecteur et un agent bactériostatique ; plus préférablement, l'activateur d'absorption muqueuse est un ou plusieurs choisis dans le groupe constitué par les tensioactifs, les cyclodextrines et leurs dérivés, le mannitol, les phospholipides, les peptides et les inhibiteurs d'enzymes protéolytiques, l'acide glycyrrhétique et ses dérivés et les agents chélateurs d'ions métalliques, et est plus préférablement le mannitol ; l'agent protecteur est un ou plusieurs choisis dans le groupe constitué par l'albumine humaine, le plasma artificiel, l'érythropoïétine, le facteur neurotrophique dérivé du cerveau, le facteur de croissance nerveuse, le facteur de croissance épidermique, le facteur de croissance des fibroblastes, le facteur de croissance basique des fibroblastes, le facteur de croissance analogue à l'insuline 1, la hyaluronidase, la neuréguline, le facteur inhibiteur de la leucémie, l'interleukine, la substance active de type interféron, le facteur de nécrose tumorale, le facteur de croissance gliale, le facteur de différenciation de la croissance, et la protéine liée à la croissance nerveuse, et est plus préférablement l'albumine humaine ; l'agent bactériostatique est l'un ou plusieurs choisis dans le groupe constitué par l'acide sorbique, le sorbate de potassium, l'acide benzoïque, les parabènes, et une utilisation combinée d'acide benzoïque et de sorbate de potassium, et est plus préférablement le benzoate de sodium ; de préférence, une concentration de l'activateur d'absorption muqueuse est 4-28 % (v/v), de préférence 6-14 % (v/v) ou 4-15 % (v/v), et plus préférablement 10 % (v/v) ou 5 % (v/v) ;
de préférence, une concentration de l'agent protecteur est de 0,3-2,5 % (p/v), de préférence de 0,8-1,6 % (p/v) ou de 0,3-1,0 % (p/v), et plus préférablement de 1,2 % (p/v) ou de 0,5 % (p/v) ; et
de préférence, une concentration de l'agent bactériostatique est de 0,2-0,6 % (p/v), de préférence de 0,25-0,4 % (p/v) ou de 0,45-0,6 % (p/v), et plus préférablement de 0,3 % (p/v) ou de 0,5 % (p/v).

8. Médicament destiné à être utilisé dans la prévention d'une infection à coronavirus, dans lequel le coronavirus est SARS-CoV-2, **caractérisé en ce que** le médicament est une formulation nasale et comprend l'interféron selon la revendication 1 et **en ce que** l'interféron est administré par voie nasale ; de préférence le coronavirus est infecté par gouttelette-nez-voies respiratoires ; et de préférence, le médicament comprend en outre le tampon décrit dans la revendication 6 et/ou le support ou excipient décrit dans la revendication 7.
